# EUROPEAN PATENT APPLICATION

(11) **EP 1 900 318 A1**
(43) Date of publication of application: **19.03.2008**
(21) Application number: 07012212.2
(22) Date of filing: 21.06.2007
(51) Int. Cl.: A61B 1/31

(54) **Endoscope and endoscope system**

(30) Priority: 15.09.2006 JP 2006251632
(71) Applicant: OLYMPUS MEDICAL SYSTEMS CORP., Shibuya-ku Tokyo 151-0072 (JP)
(72) Inventor: Tsumaru, Masayo, Tokyo 151-0072 (JP); Matsuo, Shigeko, Tokyo 151-0072 (JP)
(74) Representative: von Hellfeld, Axel

(57) **Abstract**

In order to provide a rotating self-propelling endoscope and an endoscope system capable of easily achieving miniaturization of an operation portion by using a small-sized motor, and configured to obtain sufficient propulsive force with a smaller motor torque, the endoscope of the invention comprises a flexible elongate endoscope insertion portion 6 insertable into a subject's body; and a flexible propulsive force generating portion rotatable on an outer circumferential side of the endoscope insertion portion and having a helically shaped portion 51 on an outer circumferential surface of the flexible propulsive force generating portion, wherein the helically shaped portion has a lead angle that is set to be in a range from not less than 9 degrees to not more than 15 degrees.

## Description

### BACKGROUND OF THE INVENTION

### 1. Field of the Invention

The present invention relates to an endoscope and an endoscope system, and more particularly, to an endoscope and an endoscope system wherein a helically shaped portion is disposed on an outer circumference of a flexible elongate tube that is insertable into a subject's body.

### 2. Description of the Related Art

Conventionally, endoscopes have been generally practically used wherein an elongate insertion portion is inserted into a body cavity to observe organs in a body cavity, and a treatment instrument inserted into a treatment instrument channel is used as needed to allow performing various curing treatments.

Such endoscopes are each provided with a bending portion that is bendable upward/downward and leftward/rightward on a distal end side of the insertion portion. This bending portion is configured to be interlocked with an operating member provided to an operation portion on a hand-side, to allow bending operations of the bending portion through predetermined operations of the operating member.

To insert an insertion portion of a thus configured endoscope into, for example, a convoluted duct in a body cavity, and more particularly, into a tube cavity forming a loop of 360 degrees such as a large intestine, the operating member of the operation portion is operated to bend the bending portion, and the insertion portion is, for example, twist-operated to be advanced in the tube cavity thereby being inserted toward a desired region for observation in the tube cavity.

However, there is a need for mastery for an operator to be able to operate to insert an insertion portion of an endoscope such as one mentioned above smoothly and in a short time period to a deep part in a convoluted large intestine and the like.

Therefore, it is concerned that, for example, an inexperienced operator lost in the inserting direction of the insertion portion may have trouble in the operation or change the way the intestine runs, during an inserting operation.

Accordingly, various proposals have conventionally been made to increase insertability of the insertion portion of the endoscope, such as one disclosed in Japanese Unexamined patent publication No. 2006-34627.

An endoscope system disclosed in the Japanese Unexamined patent publication No. 2006-34627 includes an endoscope that by rotates to obtain propulsive force. This endoscope is a rotating self-propelling endoscope including an insertion portion and a distal end member having a larger outer diameter than the insertion portion, the insertion portion and the distal end member each having an outer circumferential surface on which a helix is formed.

Such a configuration can decrease pressure applied to a tube cavity internal wall from the insertion portion and the distal end member when the insertion portion of the endoscope is inserted into a body cavity, and allows effective use of propulsive force obtained by the rotation of the helices.

However, the endoscope system disclosed in the above-described Japanese Unexamined patent publication No. 2006-34627 raises problems such as the following when, for example, the helix is not properly shaped. For example, insufficient propulsive force obtained by the helix rotation may prevent the insertion portion from being advanced to a deep part in an intestine.

Also, in order to obtain a strong propulsive force, means such as increasing helix height and rigidifying the insertion portion may be considered. In this case, for example, resultant large friction between the intestine and the helix requires a large motor driving torque to rotate the insertion portion, which results in increased motor size. This causes inhibiting miniaturization of the operation portion in which the motor is disposed. Further, increased size of the operation portion also increases the burden on a user grasping the operation portion of the endoscope in an operation or the like.

### SUMMARY OF THE INVENTION

An object of the present invention is to provide a rotating self-propelling endoscope and an endoscope system capable of easily achieving, by using a small-sized motor, miniaturization of an operation portion in which the small-sized motor is disposed, and of obtaining a sufficient propulsive force with a smaller motor torque.

An endoscope according to the present invention includes a flexible elongate endoscope insertion portion insertable into a subject's body; and a flexible propulsive force generating portion rotatable on an outer circumferential side of the endoscope insertion portion and having a helically shaped portion on an outer circumferential surface of the flexible propulsive force generating portion, the helically shaped portion having a lead angle that is set to be in a range from not less than 9 degrees to not more than 15 degrees.

An endoscope system according to the present invention includes the endoscope and a rotation device for rotating the propulsive force generating portion about a longitudinal axis.

Objects and advantages of the present invention will be more apparent from the following detailed descriptions.

The present invention can provide a rotating self-propelling endoscope and an endoscope system capable of easily achieving, by using a small-sized motor, miniaturization of an operation portion in which the small-sized motor is disposed, and of obtaining a sufficient propulsive force with a smaller motor torque.

### BRIEF DESCRIPTION OF THE DRAWINGS

FIG. 1 is an external view showing an entire configuration of an endoscope system in an embodiment of the present invention.
FIG. 2 is a cross sectional view showing a distal end portion, a bending portion, and part of a helically shaped portion of an endoscope of the endoscope system of FIG. 1.
FIG. 3 is a cross sectional view showing part of a connector cover connected with an operation-portion-side guiding tube of the endoscope of the endoscope system of FIG. 1.
FIG. 4 is a view on arrow viewed from IV direction of FIG. 3.
FIG. 5 is an enlarged view of essential parts of the helically shaped portion of the endoscope system of FIG. 1.
FIG. 6 is a top view showing an insertion portion housing case of the endoscope system of FIG. 1.
FIG. 7 is a view showing an action of the endoscope system of FIG. 1, showing a state where an insertion auxiliary instrument is inserted into a rectum from an anus of a patient.
FIG. 8 is a view showing an action of the endoscope system of FIG. 1, showing a state where an insertion portion main body inserted into a large intestine has reached a sigmoid colon.
FIG. 9 is a view showing an action of the endoscope system of FIG. 1, showing a state where the insertion portion main body inserted into the large intestine has reached near a cecum.
FIG. 10 is an enlarged view of essential parts in a modification example of the helically shaped portion in an embodiment of the present invention.
FIG. 11 is a schematic configuration view showing a schematic configuration of a measuring apparatus for measuring propulsive force of the helically shaped portion in an embodiment of the present invention, showing a case where contact between a coil and a subject (intestine) is set to be weak.
FIG. 12 is a schematic configuration view showing a schematic configuration of a measuring apparatus for measuring propulsive force of the helically shaped portion in an embodiment of the present invention, showing a case where contact between a coil and a subject (intestine) is set to be strong.
FIG. 13 is a graph showing change in efficiency value when lead angle is changed, of experimental results by an experimental device of FIGS. 11 and 12.
FIG. 14 is a graph showing change in propulsive force value (g) when lead angle is changed, of experimental results by the experimental device of FIGS. 11 and 12.
FIG. 15 is a graph showing change in motor torque value (g·cm) when lead angle is changed, of experimental results by the experimental device of FIGS. 11 and 12.

### DETAILED DESCRIPTION OF THE PREFERRED EMBODIMENTS

Referring to an embodiment illustrated in the drawings, the present invention is described below.

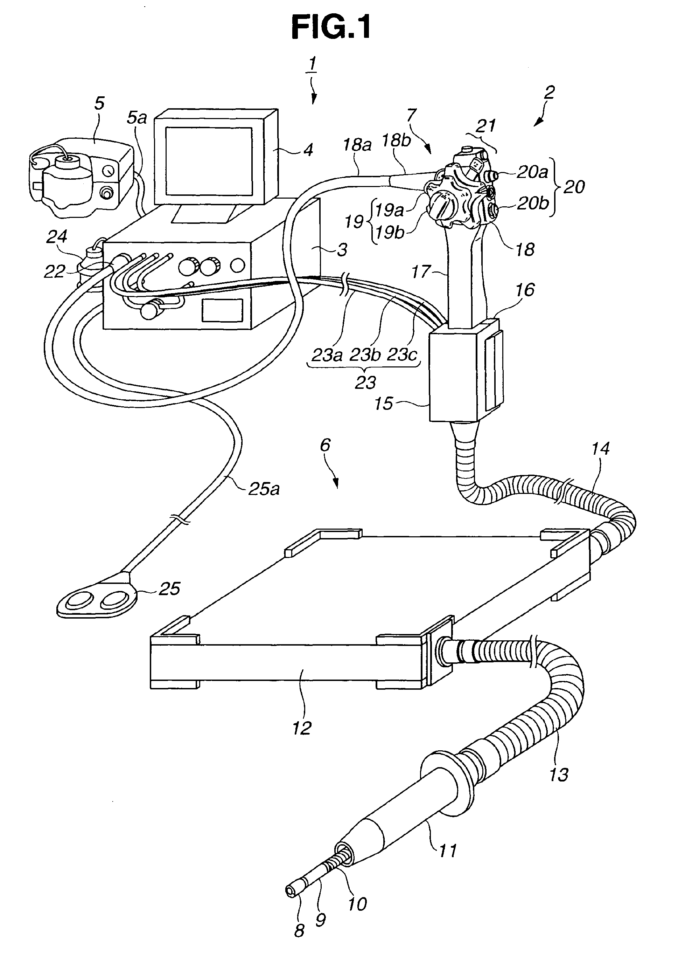

First, referring to FIG. 1, the entire configuration of an endoscope system of the present embodiment is described below. As shown in FIG 1, an endoscope system 1 of the present embodiment includes a rotating self-propelling endoscope 2.

Specifically, the endoscope system 1 is mainly configured by the rotating self-propelling endoscope 2, a controlling device 3, a monitor 4, and an aspirator 5.

The endoscope 2 is mainly configured by an insertion portion 6 and an operation portion 7. The insertion portion 6 is configured by, in the following order from a distal end side thereof, a distal end portion 8; a bending portion 9; an insertion portion main body 10; an insertion auxiliary instrument 11; an insertion portion housing case 12; a distal-end-side guiding tube 13 made of a corrugated tube interposed between the insertion auxiliary instrument 11 and the insertion portion housing case 12; an operation-portion-side guiding tube 14 made of a corrugated tube interposed between the operation portion 7 and the insertion portion housing case 12; a connector cover 15 connected with one end of the operation-portion-side guiding tube 14; and so on.

The operation portion 7 is configured by a motor box 16, a grasping portion 17, and a main operation portion 18. The motor box 16 also configures a part of the insertion portion 6. The motor box 16 incorporates a motor for applying rotation force to the insertion portion main body 10, and others.

Disposed to the main operation portion 18 are: a bending operation knob 19 for bending the bending portion 9 of the insertion portion 6 in four directions (upward/downward and leftward/rightward directions on an endoscope image captured by the endoscope 2); operation buttons 20 for fluid feeding and sucking operations; operation switches 21 used to operate an optical system relating to image pickup, illumination and the like; and others.

The bending operation knob 19 includes two operation knobs: an upward/downward bending operation knob 19a for operating the bending portion 9 in upward/downward directions on an endoscope image; and a leftward/rightward bending operation knob 19b for operating the bending portion 9 in leftward/rightward directions on an endoscope image. The operation knobs 19a, 19b are both formed in a generally disc shape. These two operation knobs are disposed on an exterior surface of the main operation portion 18 of the operation portion 7, in a coaxially superposed manner and rotatably with respect to the surface.

The upward/downward bending operation knob 19a is disposed at a position closer to the exterior surface of the main operation portion 18, and the leftward/rightward bending operation knob 19b is coaxially disposed on the upward/downward bending operation knob 19a, superposed thereon. That is, the upward/downward bending operation knob 19a is disposed at a position closer to the main operation portion 18 than the leftward/rightward bending operation knob 19b, to facilitate the upward/downward bending operation frequently performed during a normal operation of the endoscope 2.

From a side surface of the main operation portion 18 extends a universal cord 18a through which an electric cable and the like are inserted. At a root portion of the universal cord 18a from which the same extends, a folding preventing portion 18b is provided to the main operation portion 18. On a distal end side of the universal cord 18a, a connector portion 22 is disposed. The connector portion 22 is connected to a front surface of the controlling device 3.

The operation buttons 20 disposed on the exterior surface of the main operation portion 18 include: an air/water feeding button 20a to be operated to feed a gas or liquid toward the inside of the subject's body from the distal end portion 8 of the endoscope 2; and a sucking button 20b to be operated to suck body liquid or the like in the subject's body from the distal end portion 8 of the endoscope 2.

From the connector cover 15 extends three tubes 23 to be inserted in the insertion portion 6. The three tubes 23 include an air feeding tube 23a, a water feeding tube 23b, and a sucking tube 23c. Distal end sides of the three tubes 23 are respectively connected to predetermined positions on the front surface of the controlling device 3 via detachable connectors.

The controlling device 3 is detachably attached with a water tank 24 that stores therein distilled water, physiological saline or the like.

When a predetermined operation is performed of the air/water feeding button 20a of the main operation portion 18, the controlling device 3 controls to operate compressor, valves and the like not shown, to feed the distilled water, the physiological saline or the like from the water tank 24 through the water feeding tube 23b, which is spouted out from an aperture portion (not shown) formed on the distal end portion 8 toward the outside (toward front of the distal end portion 8 of the endoscope).

When the predetermined operation is performed of the air/water feeding button 20a of the main operation portion 18 of the endoscope 2, the air from the compressor is fed to the air feeding tube 23a according to operation of the compressor, the valves and the like not shown under control by the controlling device 3, to be spouted out from the predetermined aperture (not shown) formed on the distal end portion 8.

The controlling device 3 is also connected with the aspirator 5 via a tube 5a. The tube 5a is provided to continue to the sucking tube 23c connected to the front surface of the controlling device 3 via a connector.

Note that although the endoscope system 1 of the present embodiment is shown in an example using the aspirator 5 in a separate body connected to the controlling device 3, alternatively, a sucking system equipped in, for example, a hospital or facility may be used.

Then, when a predetermined operation of the sucking button 20b of the endoscope 2 is performed, the controlling device 3 controls to operate the compressor and the valves (not shown) to suck body liquid or the like in the subject's body from a sucking channel aperture (not shown) of the distal end portion 8. The sucked body liquid or the like is fed via the sucking tube 23c into the aspirator 5 connected to the controlling device 3 by the tube 5a.

Meanwhile, the controlling device 3 is connected via an electric cable 25a with a foot switch 25 used for operating to rotate in a predetermined direction and stop the insertion portion main body 10 of the endoscope 2.

Moreover, although not shown, advancing/retreating switches for rotational direction operation and stopping operation for the insertion portion main body 10, other than the foot switch 25, are also disposed on, for example, a predetermined region on an exterior surface of the main operation portion 18 of the operation portion 7, the front surface of the controlling device 3, and so on.

On the front surface of the controlling device 3 are disposed a power switch and various operating members such as an operation dial for variably operating the rotation speed the insertion portion main body 10 of the endoscope 2.

The controlling device 3 is electrically connected to the monitor 4. The monitor 4 is a display device for displaying an endoscope image obtained by the endoscope 2.

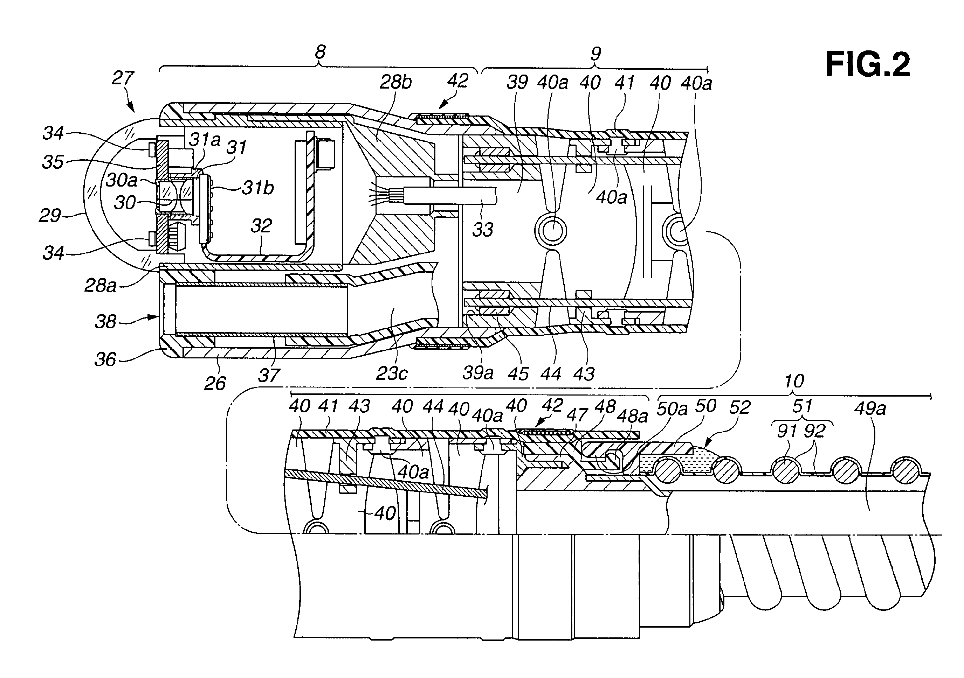

Next, referring to FIG. 2, detailed configurations of the distal end portion 8, the bending portion 9, and the insertion portion main body 10 configuring a part of the insertion portion 6 of the endoscope 2 are described below.

Firstly, the distal end portion 8 is configured by a main body tube 26 formed in a generally cylindrical shape by a biocompatible resin member. Inside the main body tube 26 are disposed an image pickup unit 27 and others. The image pickup unit 27 has an external shape formed by a holding tube 28a in a generally short tubular shape; a cover tube 28b disposed to cover a proximal end surface and a part of an outer circumference of the holding tube 28a; and a cover body 29 in a dome shape disposed to cover a front surface side of the holding tube 28a.

The holding tube 28a and the cover tube 28b are formed of a biocompatible metal. The cover body 29 is formed of a biocompatible transparent synthetic resin. The holding tube 28a is accommodated inside the main body tube 26.

The cover tube 28b is fitted on a proximal end side of the holding tube 28a and has a bottom surface portion formed with a through hole through which a signal cable 33 is inserted.

The cover body 29 is fitted to airtightly seal a distal end aperture of the front surface side of the holding tube 28a.

In the image pickup unit 27, a space is formed by the holding tube 28a, the cover tube 28b, the cover body 29, and the like. In the space, there are disposed a group of objective lenses 30; an image pickup device 31 which is a photoelectric conversion device such as CCD (Charge Coupled Device) and CMOS (Complementary Metal Oxide Semiconductor) disposed on an optical axis of the group of objective lenses 30; a flexible print substrate 32; and so forth.

To the flexible print substrate 32, there are mounted a circuit for performing various signal processings such as amplification in response to an image signal generated by photoelectric conversion processing by the image pickup device 31, and others. The flexible print substrate 32 is connected with the signal cable 33. The signal cable 33 extends from the through hole of the cover tube 28b, inserted from the distal end portion 8 to the bending portion 9 and the insertion portion main body 10, leading to the connector cover 15 (see FIG. 1), to be connected to a connector (not shown) inside the connector cover 15.

The group of objective lenses 30 is held by an objective lens barrel 30a. The objective lens barrel 30a is fixed to a holding body 35. To the rear of the objective lens barrel 30a is fitted an image pickup device holding frame 31a to hold the image pickup device 31.

On a rear surface of the image pickup device 31 is mounted a circuit substrate 31b, which is electrically connected with the flexible print substrate 32.

The holding body 35 is formed in a generally circular shape, and has a periphery portion fixed to an inner circumferential surface on the proximal end side of the cover body 29. At this time, the holding body 35 is disposed with respect to the cover body 29 so that a center axis of the cover body 29 generally agrees with an optical axis of the group of objective lenses 30.

To the holding body 35, a plurality of LEDs 34 serving as illumination portions are also disposed to surround the group of objective lenses 30.

The image pickup unit 27 thus configured is fixed to the main body tube 26 by a distal end cap 36 disposed at the aperture portion on the distal end side of the main body tube 26, in a state disposed at a predetermined position decentered from a longitudinally directed center axis of the main body tube 26.

Between the holding tube 28a of the image pickup unit 27 and the main body tube 26 is formed a gap, in which are disposed a distal end part of the sucking tube 23c and a sucking tube 37 continuously provided on a distal end side of the sucking tube 23c. A distal end part of the sucking tube 37 is fixed to the distal end cap 36.

On a distal end side of the distal end cap 36 is formed a channel aperture portion 38 that is open to the front.

Though not shown, in the gap formed between the holding tube 28a and the main body tube 26, there are also disposed tubes communicating with the air feeding tube 23a and the water feeding tube 23b, and the distal end cap 36 is formed with channel aperture portions of the tubes 23a, 23b.

Next, the bending portion 9 is described. In the bending portion 9, there are continuously and rotatably provided a rigid distal end bending piece 39 fitted to a proximal end aperture portion of the main body tube 26 configuring the distal end portion 8 and a plurality of rigid bending pieces 40 (also referred to as bending joint rings), by means of a pivot portion 40a. The bending pieces 39, 40 are coated by a bending cover 41 formed of a biocompatible elastic member such as a fluoro rubber. A distal end part of the bending cover 41 is fastened to a proximal end portion of the main body tube 26 by means of a thread-wound adhering portion 42 on an outer circumferential side of the bending cover 41.

Through the plurality of bending pieces 40 are inserted four bending operation wires 44 (only two wires are shown in FIG. 2) that are inserted in through the insertion portion main body 10. On respective inner circumferential surfaces of the plurality of bending pieces 40, there are inwardly protruded wire guides 43 for holding respective distal end sides of the four bending operation wires 44. The four bending operation wires 44 are each inserted through the wire guides 43, and tubular engaging members 45 soldered and fixed to distal end portions of the four bending operation wires 44 are respectively engaged with four engaging hole portions 39a formed on the distal end bending piece 39.

The four engaging hole portions 39a are formed at positions quartered at generally equal intervals on a surface orthogonal to a longitudinally directed axis of the distal end bending piece 39. The distal end bending piece 39 has a circum-axial direction determined such that the engaging hole portions 39a are positioned to respectively correspond to upward/downward and leftward/rightward directions on an endoscope image. Therefore, the four bending operation wires 44 are held and fixed at four points separated at generally equal intervals in upward/downward and leftward/rightward directions.

The four bending operation wires 44 are also coated by coil pipes not shown and are inserted in through the insertion portion main body 10, to extend to the connector cover 15.

To proximal end portions of the bending operation wires 44 are provided with wire hooks not shown. The wire hooks of the bending operation wires 44 are respectively connected to wire connecting members not shown provided in the grasping portion 17, in a state where the connector cover 15 is united with the motor box 16.

Here, the wire connecting members are respectively connected to a bending operation function and chains (not shown) disposed in the main operation portion 18 and interlocked with the bending operation knob 19. That is, when the bending operation knob 19 of the endoscope 2 is rotated and operated, the wire connecting members are alternately pulled or relaxed using the bending operation function. This causes the bending operation wires 44 to be pulled or relaxed.

Therefore, in the endoscope 2, pulling or relaxing each of the four bending operation wires 44 orients the distal end bending piece 39 in one of upward/downward and leftward/rightward directions, which at the same time causes the plurality of bending pieces 40 to move to follow the distal end bending piece 39, to bend and operate the bending portion 9 in one of upward/downward and leftward/rightward directions.

To a proximal end portion of the bending portion 9, there are disposed an internal-layer tube base 47 made of a metal member for fixing an internal-layer tube, which is fitted on an outer circumferential side of the bending pieces 40 disposed at the proximal-most end; and a helical tube connecting base 48 made of a metal member fitted on an outer circumferential side of the internal layer tube base 47 and rotatably engaging with a helically shaped portion (helical tube) 51 which is a rotating cylindrical body to be described later. The bases 47, 48 are tightly fastened to each other with an adhesive or the like.

On an outer circumferential side of the helical tube connecting base 48, a proximal end portion of the bending cover 41 is coveringly disposed. In this region, the bending cover 41 is fixed to the helical tube connecting base 48 from an outer circumferential side by means of the thread-wound adhering portion 42.

A proximal end portion of the internal layer tube base 47 is fixed to a distal end part of a flexible internal-layer tube 49a inserted through the insertion portion main body 10.

A proximal end part of the helical tube connecting base 48 is provided with a protruding portion 48a with a snap-fit shape. The protruding portion 48a engages with an engaging portion 50a of a distal-end-side base 50 provided to a distal end portion of the insertion portion main body 10 to be described later.

Next, the insertion portion main body 10 is described. The insertion portion main body 10 is configured by the distal-end-side base 50 provided to a distal end part thereof, formed of a synthetic resin, and serving as an engaging portion for connection with the bending portion 9; and a helically shaped portion 51 having a distal end part fixed to the distal-end-side base 50 by an adhesive 52.

In the insertion portion main body 10, the internal-layer tube 49a is disposed. The internal-layer tube 49a is formed by a tube body or the like made of a thin wire or the like flexibly woven in a tubular shape. In the internal-layer tube 49a are inserted and disposed the bending operation wires 44, the signal cable 33, the power cable (not shown) to the LEDs 34, and the tubes 23 such as the air feeding tube 23a. The internal-layer tube 49a thus protects the above-described internally inserted and disposed components.

To a distal end part of the distal-end-side base 50, there is formed the engaging portion 50a for engaging with the protruding portion 48a of the helical tube connecting base 48 to activate the snap-fit function. In a state where the engaging portion 50a of the distal-end-side base 50 is engaged with the protruding portion 48a of the helical tube connecting base 48, the distal-end-side base 50 is rotatable with respect to the helical tube connecting base 48 about a longitudinally directed axis. A distal end outer circumference of the distal-end-side base 50 is covered by a proximal end portion of the bending cover 41 by a small clearance.

The helically shaped portion 51 unitedly fixed to the distal-end-side base 50 by the adhesive 52 is rotatable about the longitudinally directed axis as an inserting direction.

The helically shaped portion 51 is configured by a coil 91 and a thin resin coat 92, and is provided over a range of not less than 600 mm from the distal end part of the insertion portion main body 10 or over the entire length of the insertion portion main body 10. The reason for the setting of the helically shaped portion 51 being provided over a range of not less than 600 mm from the distal end part of the insertion portion main body 10 is based on that the length from the anus to the boundary between a sigmoid colon and a descending colon is generally said to be about 600 mm. In a case where the helically shaped portion 51 is not provided over the entire length of the insertion portion main body 10, parts that are not the helically shaped portion 51 are configured by a flexible tube, for example.

The helically shaped portion 51 is applied with a rotation force by a motor (not shown) disposed in the motor box 16 (see FIG. 1) of the operation portion 7. When applied with a rotation force by the motor, the helically shaped portion 51 rotates in contact with a body cavity internal wall of the subject's body, thereby generating a propulsive force, which causes the helically shaped portion 51 to be advanced in the inserting direction. At this time, the distal-end-side base 50 fixed to the distal end portion of the helically shaped portion 51 contacts the helical tube connecting base 48 to push the bending portion 9. This causes the entirety of the insertion portion main body 10 including the distal end portion 8 to advance toward a deep part in the body cavity.

Thus, the motor box 16 incorporating the motor functions as a rotation device for rotating the helically shaped portion 51 (propulsive force generating portion to be described later) about a longitudinal axis.

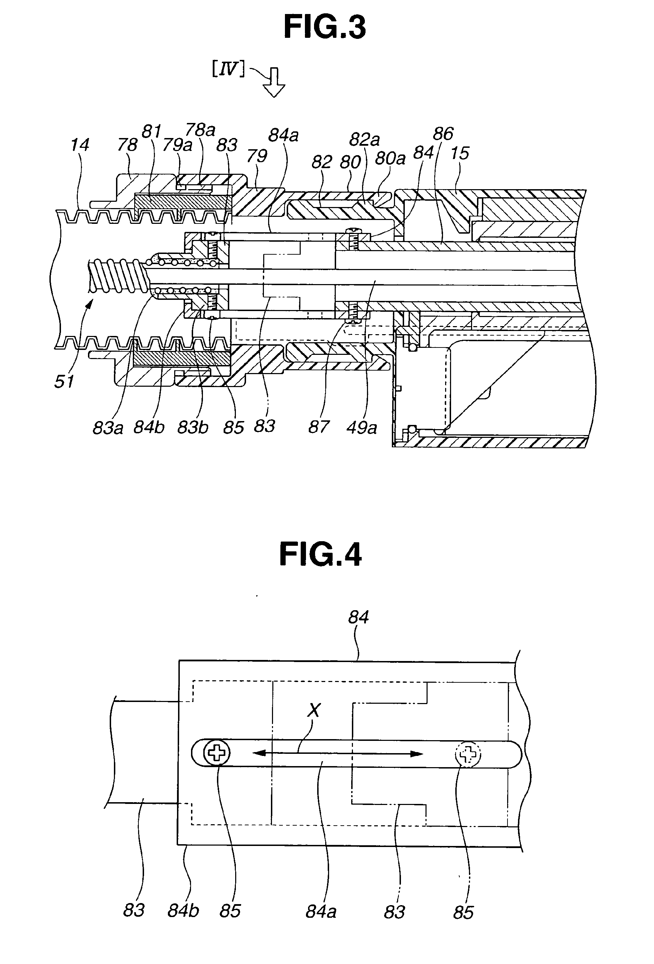

Next, referring to FIGS. 3 and 4, configuration of a proximal end side of the helically shaped portion 51 is described below. First, connecting part between the operation-portion-side guiding tube 14 and the connector cover 15 is described.

On an outer circumferential side of a proximal end portion of the operation-portion-side guiding tube 14, a joint ring 81 is screwed. On an outer circumferential side of the joint ring 81, a fifth fixing annulus 78 formed of a generally cylindrically shaped metal member (which may also be formed of a rigid cylindrical body formed of a synthetic resin, plastic, or the like) and a connecting cylindrical body 79 formed of a synthetic resin are screwed and connected to each other. This causes the joint ring 81 to be fit and held in the fifth fixing annulus 78 and the connecting cylindrical body 79.

Connection between the fifth fixing annulus 78 and the connecting cylindrical body 79 is as follows. That is, the fifth fixing annulus 78 is a cylindrical body formed such that an aperture at one end portion is smaller than an aperture at the other (proximal end side) end portion. In other words, a half-way portion of the fifth fixing annulus 78 is shaped to protrude toward an outer diameter direction. In this case, the aperture at the one end portion has a diameter set to be generally the same as an outer diameter of the operation-portion-side guiding tube 14, the aperture at the other end portion has an inner diameter set to be generally the same as an outer diameter of the joint ring 81, and the aperture of the other end portion has an outer diameter set to be generally the same as an inner diameter of one end portion of the connecting cylindrical body 79. On an outer circumferential surface on a proximal end side of the fifth fixing annulus 78, a male screw portion78a is formed.

On the other hand, the connecting cylindrical body 79 is a cylindrical body formed such that an aperture on one end portion is larger than an aperture on the other (proximal end side) end portion. That is, a distal end part of the connecting cylindrical body 79 is shaped to protrude toward an outer diameter direction. In this case, the one end portion has an aperture with a diameter that is set to be generally the same as an outer diameter of the proximal end portion of the fifth fixing annulus 78. On an inner circumferential surface at a distal end part of the connecting cylindrical body 79, a female screw portion 79a is formed.

To the other end portion of the connecting cylindrical body 79 are formed engaging portions 80 to allow a detachable engagement with the connector cover 15. The engaging portions 80 each have an end portion that is formed with an engaging nail 80a.

With such a configuration, the fifth fixing annulus 78 and the connecting cylindrical body 79 are connected to each other by means of screwing between the male screw portion 78a and the female screw portion 79a. At this time, the joint ring 81 is fit and held in the inside of the connection portion of the male screw portion 78a and the female screw portion 79a.

Under this state, the proximal end portion of the operation-portion-side guiding tube 14 is in a compressed state, with a proximal end outer circumferential portion being pushed to an inside end surface of the connecting cylindrical body 79. This causes the operation-portion-side guiding tube 14, the fifth fixing annulus 78, and the connecting cylindrical body 79 to be watertightly connected to one another.

The engaging portions 80 of the connecting cylindrical body 79 are engaged with connecting portions 82 of the connector cover 15, to connect the operation-portion-side guiding tube 14 and the connector cover 15 to each other.

In more detail, distal and proximal end parts of the connector cover 15 each have the cylindrically shaped connecting portions 82. On an outer circumferential surface of the connecting portion 82, a protruding nail portion 82a is formed.

The connecting portion 82 is externally fitted and connected with the plurality of engaging portion 80 of the connecting cylindrical body 79. To the end portion on the proximal end side of each of the plurality of the engaging portion 80, the engaging nail 80a is formed as mentioned above. The engaging nail 80a is formed having an inward protrusion.

Thus, the connection between the connecting cylindrical body 79 and the connector cover 15 is performed by inserting and disposing the connecting portion 82 inside the engaging portion 80 thus bringing about engagement between the engaging nail 80a of the engaging portion 80 and the protruding nail portion 82a of the connecting portion 82. Furthermore, since the engaging portion 80 and the connecting portion 82 are both elastic, pulling the engaging portion 80 out from the connecting portion 82 can cancel the engagement between the engaging nail 80a and the protruding nail portion 82a, thereby detaching the connecting cylindrical body 79 from the connector cover 15.

Also, when the engaging nail 80a of the engaging portion 80 and the protruding nail portion 82a of the connecting portion 82 are engaged to each other, the connecting cylindrical body 79 is rotatable about an axis, with respect to the connector cover 15. Accordingly, the operation-portion-side guiding tube 14 connected to the connecting cylindrical body 79 is also rotatable with respect to the connector cover 15.

Inside of the thus configured connecting part between the operation-portion-side guiding tube 14 and the connector cover 15, a proximal end portion of the helically shaped portion 51 is fixed to a proximal end side base 83 by an adhesive 83a. The proximal end side base 83 is fitted by insertion in a slide cylinder 84. In two opposing surfaces of the slide cylinder 84 are symmetrically formed two long holes 84a in which head portions of male screws 85 are respectively fitted.

The proximal end side base 83 has female screw portions 83b formed at positions corresponding to the long holes 84a of the slide cylinder 84, to which the male screws 85 are to be screwed. A proximal end side of the slide cylinder 84 is connected to a distal end part of a rotation shaft 86 by fixing screws 87.

On a distal end side of the slide cylinder 84, a flange portion 84b is formed to prevent the proximal end side base 83 from being pulled off.

Here, as shown in FIG. 4, the proximal end side base 83 is slidable in a longitudinal direction (arrow X direction in FIG. 4) between the flange portion 84b and a distal end side of the rotation shaft 86 (see FIG. 3, not shown in FIG. 4). Also, though not shown, the rotation shaft 86 is rotatably supported in the connector cover 15.

Thus, even if applied with a torque while rotating, the helically shaped portion 51 can expand and contract in a longitudinal direction thereby preventing hardening thereof owing to the sliding of the proximal end side base 83, which prevents insertability of the helically shaped portion 51 from decreasing.

The endoscope 2 is configured such that, when the connector cover 15 is connected to the motor box 16 (see FIG. 1), a gear (not shown) provided to the rotation shaft 86 and a gear (not shown) provided in the motor box 16 engage to each other, thereby transmitting a driving force of the motor to each of the gears to rotate the helically shaped portion 51 about a longitudinal axis via the rotation shaft 86 and the proximal end side base 83. In other words, the helically shaped portion 51 transmits, from a proximal end portion thereof, a rotating driving force from the motor box 16.

Note that the internal-layer tube 49a inserted through the helically shaped portion 51 leads from inside the connector cover 15 through the rotation shaft 86 to the helically shaped portion 51.

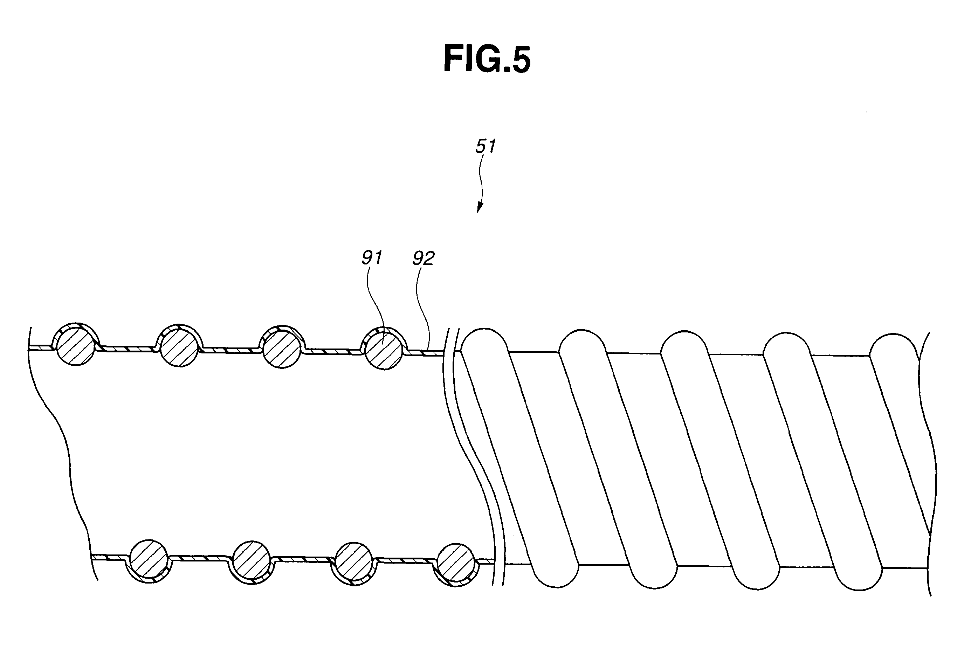

Next, referring to FIG. 5, detailed configuration of the helically shaped portion 51 is described below.

The helically shaped portion 51 is disposed to be rotatable coaxially with the internal-layer tube 49a, on an outer circumferential side of the internal-layer tube 49a, to function as a propulsive force generating portion. The helically shaped portion 51 is formed by the coil 91 which is biocompatible and loosely wound and the thin resin coat 92 which is biocompatible and provided to link respective intervals between adjacent threads of the coil 91.

Applied as a material of the coil 91 is, for example, a metal member such as a Ni- (nickel) free coil, or a resin member. The wire of the coil 91 has a cross section in, for example, a generally circular shape, and has a diameter set to be about, for example, 1.0 mm to provide a good torque traceability. The coil 91 has a lead angle that is set in, for example, a range from not less than 9 degrees to not more than 15 degrees to allow a preferable propulsive speed for endoscopy.

As shown in FIG. 5, the thin resin coat 92 is disposed in a form to coat an outer circumferential side of the coil 91 in a manner linking the respective intervals between the adjacent threads of the coil 91. This brings the respective intervals between the threads of the coil 91 into a linked arrangement.

The thin resin coat 92 is formed by a material having, for example, a hardness of 50 to 90 degrees and thickness of 0.03 to 0.2 mm, in consideration of balance between pliablity and endurance. As a resin to form the thin resin coat 92 is used a biocompatible resin member having good slidability, pliablity and formability of, for example, urethane, thermoplastic resin, polyester, or the like, and is formed to be transparent or semitransparent or in a dark color.

In this manner, the thin resin coat 92 coats the outer circumferential side of the coil 91 while linking the respective intervals between the adjacent threads of the coil 91, which allows highly forming protrusions of the helically shaped portion 51. This provides a characteristic of proving a good catch with respect to the body cavity internal wall, generating a strong propulsive force. Also, use of the metal coil 91 gives the helically shaped portion 51 advantages that helix angle (lead angle) or the like of the coil can be designed to be formed as desired, and further that configuration of the helically shaped portion 51 is prevented from becoming complex.

Furthermore, use of the loosely wound coil 91 also provides an advantage of allowing lightweight configuration, thus maintaining a good operatability of the insertion portion main body 10.

In addition, in the helically shaped portion 51, the thin resin coat 92 does not inwardly protrude from an inner circumferential side of the metal coil 91 thus not interfering with the internal-layer tube 49a, which also effectively enables the helically shaped portion 51 to be surely fixed to the proximal end side base 83.

Note that, when the helically shaped portion 51 is bent to the maximum limit, it is considered possible the thin resin coat 92 serving as a coating member of the coil 91 may inwardly protrude from the intervals between the threads of the coil 91. In such a case, in order to prevent the thin resin coat 92 from interfering with or pushing internally disposed members such as the internal-layer tube 49a, the interference causing co-rotation of the internal-layer tube 49a, a sufficient clearance is provided between the thin resin coat 92 and the internal-layer tube 49a.

Meanwhile, the helically shaped portion 51, which is formed using the metal coil 91, is expandable and contractable. This brings about an effect of, when, for example, a distal end of the insertion portion strikes an intestine wall, mildly changing a force with which the distal end pushes the intestine, thus reducing load on the intestine.

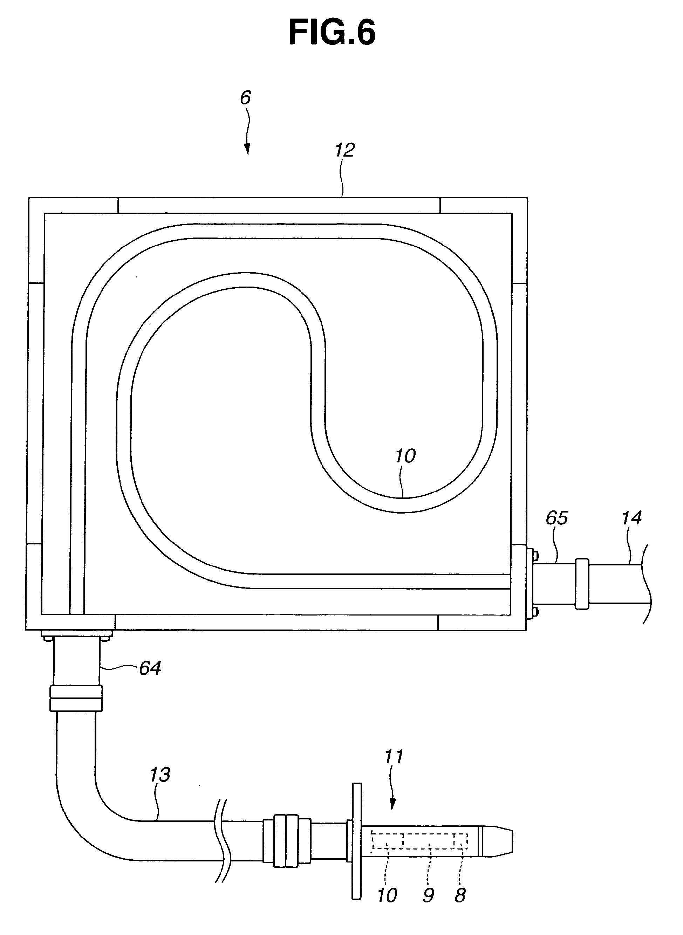

In this manner, the insertion portion 6 of the endoscope system 1 in the present embodiment is configured by components such as the distal end portion 8, the bending portion 9, the insertion portion main body 10, the insertion auxiliary instrument 11 covering these components, the distal-end-side guiding tube 13, the insertion portion housing case 12, the operation-portion-side guiding tube 14 (see FIGS. 1 and 6).

Note that the distal-end-side guiding tube 13 and the insertion portion housing case 12 are connected via a guiding tube fixing member 64. Inside the guiding tube fixing member 64 is provided a function (not shown) that uses rotation force applied to the helically shaped portion 51 by causing a rubber plate or the like to fit with the helically shaped portion 51, to give propulsive force to the helically shaped portion 51.

The operation-portion-side guiding tube 14 and the insertion portion housing case 12 are connected via a guiding tube fixing member 65.

The insertion portion 6, which is configured to be disposably used after each use, may be used in a form of being sufficiently sterilized and disinfected after each use and reused.

Referring to FIGS. 6 to 9, actions of the thus configured endoscope system 1 in the present embodiment are described below. The following description is made taking an exemplary case of performing large intestine endoscopy using the endoscope system 1 of the present embodiment.

Basic usage configuration of the endoscope system 1 is as shown in FIG. 1. At this time, the insertion portion main body 10 is housed in the insertion portion housing case 12, in a configuration as shown in FIG. 6, a looped state, for example.

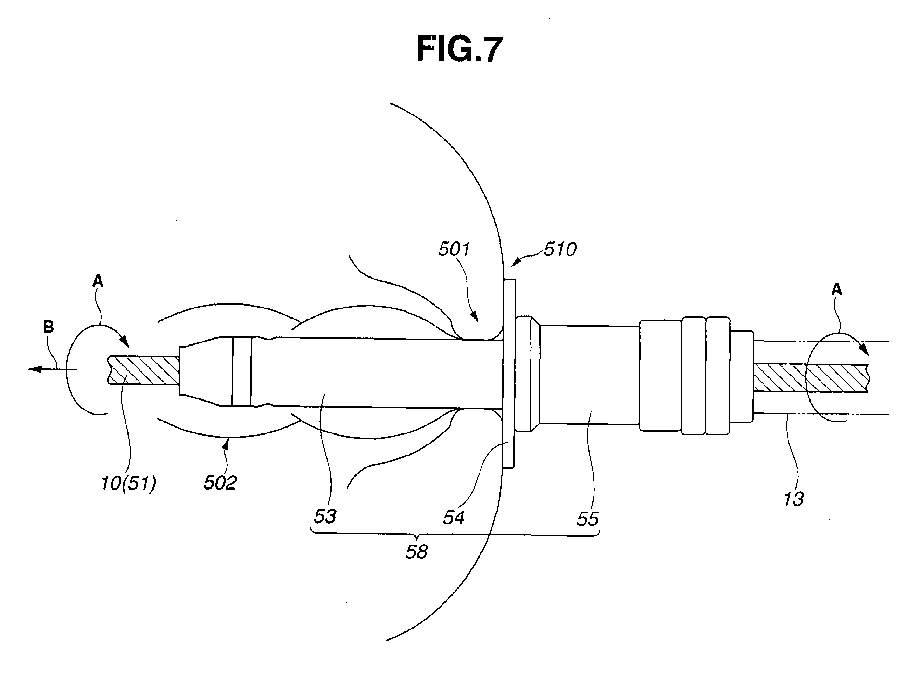

First, an operator inserts the insertion auxiliary instrument 11 of the endoscope system 1 from an anus 501 (see FIG. 7) of a patient lying on a bed, for example.

The insertion auxiliary instrument 11 is then brought into a state where only an insertion tube 53 is inserted into a rectum 502 from the anus 501, with a contacting portion 54 being in contact with a buttock 510 near the anus 501 of the patient as shown in FIG. 7. That is, the contacting portion 54 prevents the insertion auxiliary instrument 11 from being entirely inserted into the rectum 502.

When the insertion auxiliary instrument 11 is brought into a state as shown in FIG. 7, the operator fixes the contacting portion 54 to the buttock 510 of the patient with a tape or the like.

In this state, the operator grasps the grasping portion 17 of the operation portion 7, and then performs a predetermined operation such as foot operation of the foot switch 25 or hand operation of the advancing/retreating switch provided to the main operation portion 18, to rotate the helically shaped portion 51 of the insertion portion main body 10 in a direction (arrow direction A shown in FIG. 7) that is a predetermined direction about the longitudinal axis, in which the helically shaped portion 51 is advanced and the helically shaped portion 51 is inserted into the body cavity.

In other words, the operator makes the motor disposed in the motor box 16 of the operation portion 7 rotatably drivable by the foot operation or the hand operation. A rotation force is transmitted from a proximal end portion to a distal end side of the helically shaped portion 51, which is thereby entirely rotated in the direction about an axis shown in the arrow A in FIG. 7 to be applied with propulsive force from the guiding tube fixing member 64 of the insertion portion housing case 12.

The helically shaped portion 51 applied with the propulsive force causes the distal-end-side base 50 shown in FIG. 2 to push the helical tube connecting base 48. This makes the entirety of the insertion portion main body 10 including the distal end portion 8 and the bending portion 9 advance toward a deep part in the large intestine (along a direction of an arrow B in FIG. 7) via the distal-end-side guiding tube 13 and the insertion auxiliary instrument 11.

At this time, the operator is only required to lightly grasp a holding tube 55 of the insertion auxiliary instrument 11, without grasping and pushing to advance the insertion portion main body 10. The insertion portion main body 10 is thus advanced into the deep part in the large intestine only by the propulsive force applied in the guiding tube fixing member 64.

The advancing of the insertion portion main body 10 by the action of the helically shaped portion 51 results from that contact state between the helically shaped portion 51 inserted in the intestine and folds of the intestine wall is relation between male and female screws.

In other words, the helically shaped portion 51 is smoothly advanced by the propulsive force applied in the guiding tube fixing member 64 and a propulsive force generated by contact with folds of the intestine wall, which resultantly advances the insertion portion main body 10 from the rectum 502 toward a deep part of a sigmoid colon 503.

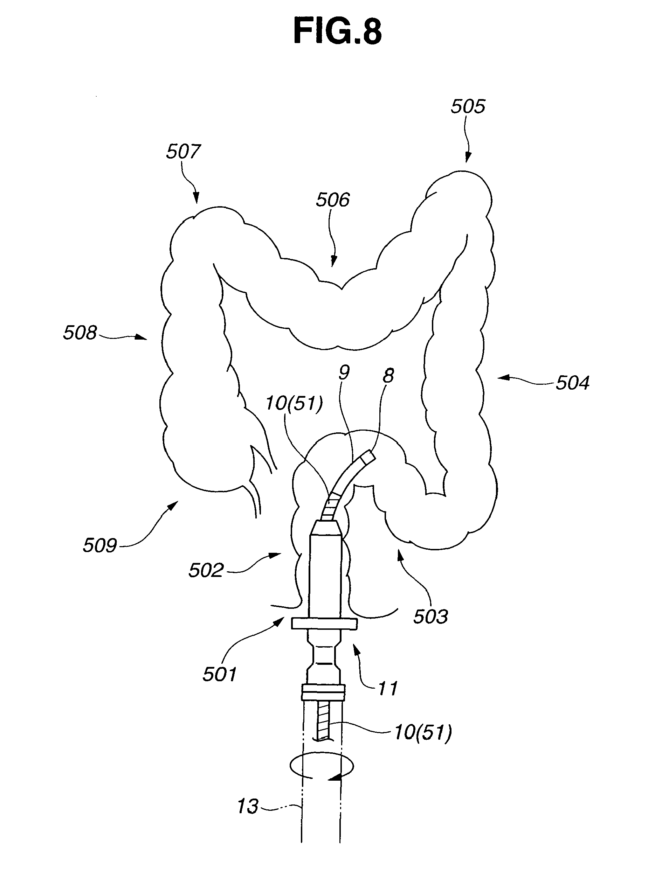

In this manner, the insertion portion main body 10 has the distal end portion 8 and the bending portion 9 reach the sigmoid colon 503 as shown in FIG. 8. At this time, the operator operates the bending operation knob 19 of the main operation portion 18 (see FIG. 1) viewing an endoscope image displayed by the monitor 4, to bend the bending portion 9 according to bending state of the sigmoid colon 503.

Through the bending operation of the bending portion 9, the operator can smoothly pass the distal end portion 8 through the sigmoid colon 503 insertion into which is difficult, while advancing the distal end portion 8 by means of the insertion portion main body 10 applied with propulsive force. The insertion portion main body 10, which is always applied with a propulsive force in the guiding tube fixing member 64, has a greater length of contact between the helically shaped portion 51 and the intestine wall as being inserted into deeper part in the large intestine.

For this reason, the insertion portion main body 10 can always obtain a stable propulsive force in a direction toward the deep part in the large intestine, whether a part of the helically shaped portion 51 is in contact with folds of the sigmoid colon 503 or the insertion portion main body 10 is convolutedly bent. The insertion portion main body 10, which is sufficiently flexible, is smoothly advanced along the intestine wall without changing the way the sigmoid colon 503 subject to easy positional change runs.

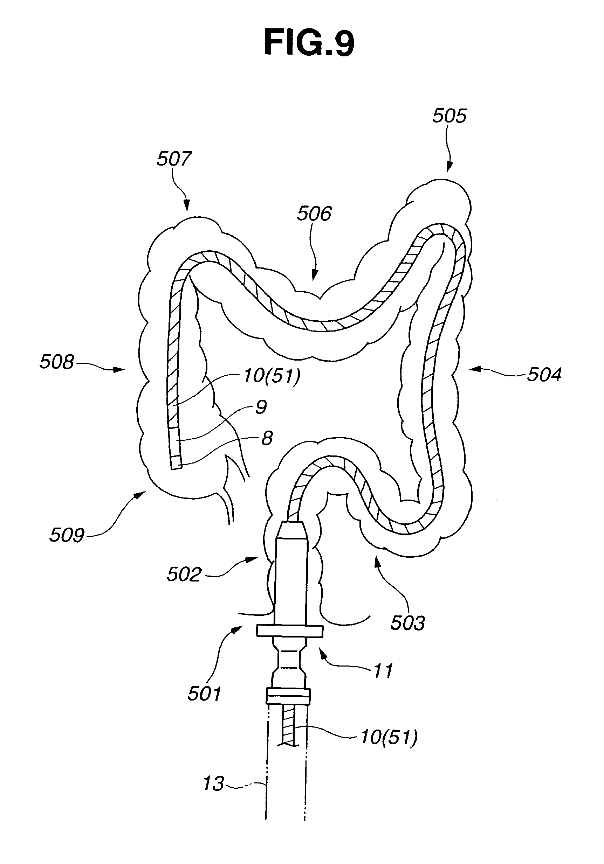

Then, the insertion portion main body 10 passes, in the following order, the sigmoid colon 503; a flexion as a boundary between the sigmoid colon 503 and a descending colon 504 which is poorly movable; the descending colon 504; a splenic flexure 505 as a boundary between the descending colon 504 and a transverse colon 506 which is highly movable; the transverse colon 506; a hepatic flexure 507 as a boundary between the transverse colon 506 and the ascending colon 508; and an ascending colon 508. Thereafter, the insertion portion main body 10 reaches, for example, near a cecum 509 as a destination region as shown in FIG. 9. During the advancing toward the region, the insertion portion main body 10 smoothly advances along intestine walls without changing general state of the large intestine.

During this inserting operation, when the distal end portion 8 has reached the flexures (the splenic flexure 505 and the hepatic flexure 507), the operator performs bending operation in accordance with bending states of respective regions by operating the bending operation knob 19 of the main operation portion 18, while viewing an endoscope image displayed by the monitor 4 in the same manner as mentioned above.

The operator, after judging from the endoscope image on the monitor 4 that the distal end portion 8 has reached near the cecum 509, once stops the rotation of the helically shaped portion 51 by the foot operation or the hand operation.

Next, the operator performs the foot operation of the foot switch 25 or the hand operation of the advancing/retreating switch of the main operation portion 18 to rotate the helically shaped portion 51 in a direction reverse to the rotation direction thereof about the axis in insertion.

That is, the operator inspects the large intestine, while rotating the helically shaped portion 51 in a direction reverse to that in insertion to retreat the insertion portion main body 10 in a direction of extracting the distal end portion 8 from the deep part of the large intestine and near the cecum 509. At this time, the operator can retreat the insertion portion main body 10 by means of a retreating force applied to the helically shaped portion 51 in the guiding tube fixing member 64, without touching the insertion portion main body 10 by the hand. Furthermore, the entirety of the insertion portion main body 10 is retreated by the propulsive force of the helically shaped portion 51, with the distal end portion 8 and the bending portion 9 being pulled by the helically shaped portion 51 through the snap-fit function.

When the distal end portion 8 of the insertion portion main body 10 has reached the insertion auxiliary instrument 11, the operator extracts the insertion portion main body 10 from the anus 501 of the patient together with the insertion auxiliary instrument 11 and completes the large intestine inspection. At this time, the insertion portion main body 10 is applied with a retreating force in the guiding tube fixing member 64 and thereafter housed in the insertion portion housing case 12 in the original state as shown in FIG. 6.

As describe above, the endoscope system 1 of the present embodiment is configured to provide an excellent insertability with which the insertion portion main body 10 can be easily inserted into the deep part of the large intestine. Also, in the endoscope 2 of the present embodiment, the guiding tubes 13, 14 for coupling the insertion portion housing case 12 with the insertion auxiliary instrument 11 and the operation portion 7, respectively, is pliably flexible. Therefore, with the endoscope 2 of the present embodiment, even fixedly mounting the insertion portion housing case 12 does not limit position of the operation portion 7 grasped by the operator and position of the insertion auxiliary instrument 11 approaching the anus of the patient, which allows moving these units to a desired position in a predetermined allowed area.

In other words, in the endoscope 2 of the present embodiment, because the distal-end-side guiding tube 13 connecting the insertion auxiliary instrument 11 and the insertion portion housing case 12 is a pliable tube body, it is not necessary to keep constant the positional relation between the anus of the patient and the insertion portion housing case 12. Also, in the endoscope 2 of the present embodiment, pliability of the operation-portion-side guiding tube 14 prevents the operation portion 7 from being limited in degree of freedom of motion.

Meanwhile, because the insertion portion housing case 12 and the guiding tubes 13, 14 are formed of a transparent or semitransparent material, motion of the insertion portion main body 10, especially rotation state of the helically shaped portion 51, can be visually checked.

Moreover, in the endoscope 2 of the present embodiment, connecting portions for the insertion auxiliary instrument 11, the distal-end-side guiding tube 13, the insertion portion housing case 12, and the operation-portion-side guiding tube 14 are watertightly held. Therefore, the endoscope 2 of the present embodiment can prevent liquid such as large intestine drainage from spattering in an operation room. Thus, the insertion portion 6 has a sanitarily excellent structure.

Also, the insertion auxiliary instrument 11 prevents the insertion portion main body 10 before insertion into a body cavity from being subject to a resistance caused by tightening or the like of the anus 501 of the patient, which can reduce occurence of deflection and preventing twisting due to rotation.

Furthermore, the insertion auxiliary instrument 11 prevents the insertion portion main body 10 during introduction into the large intestine from directly contacting the anus 501. Therefore, the highly flexible insertion portion main body 10 is free from a resistance such as by tightening of the anus 501, which improves introducability into the large intestine.

As a result, the endoscope 2 of the present embodiment and the insertion portion 6 thereof are configured such that the insertion portion main body 10 and the helically shaped portion 51 before insertion into the subject's body can be smoothly inserted into the subject's body, thus achieving high operatability.

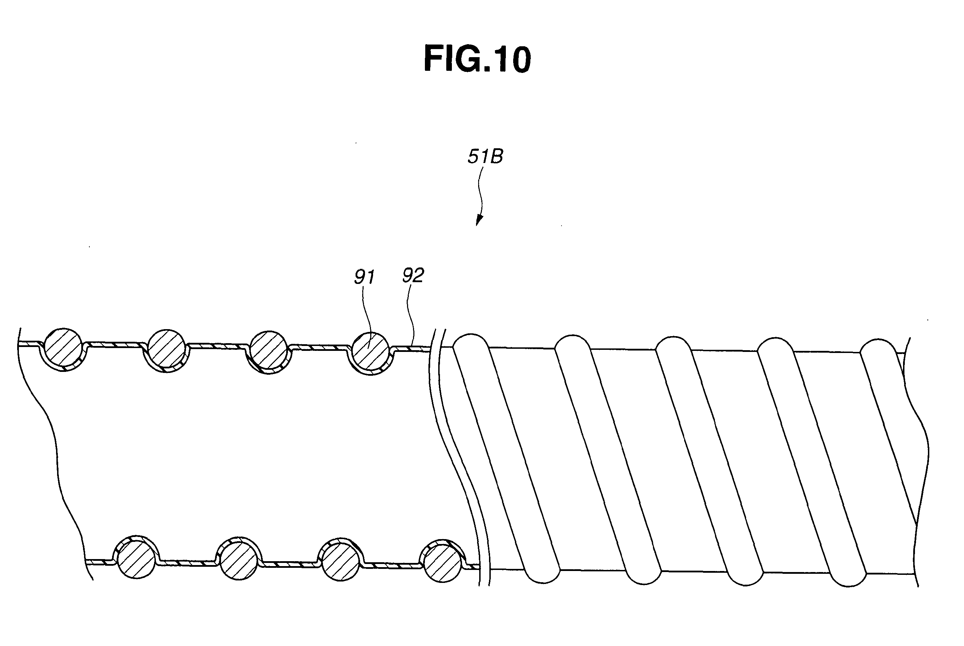

Note that the helically shaped portion 51 described in the above-described embodiment, which is configured to cover the outer circumference of the coil 91 to link the respective intervals between the threads of the coil 91, may be configured as shown in FIG. 10.

FIG. 10 is an enlarged view of essential parts of a modification example of the helically shaped portion in FIG. 5. Note that FIG. 10 shows a part of the helically shaped portion in a section view.

As shown in FIG. 10, in a helically shaped portion 51B in this modification example, the thin resin coat 92 disposed to link the respective intervals between the threads of the coil 91 is disposed to coat an inner circumferential side of the coil 91.

This allows the helically shaped portion 51B in the present modification example to have an increased slidability in a rotation direction compared to the case with the helically shaped portion 51 in the above-described embodiment, which provides an effect of decreasing the load on the motor for generating a rotating driving force.

Meanwhile, although in the above-described embodiment, the rotating driving force of the motor incorporated in the motor box 16 is transmitted to the proximal end side of the helically shaped portion 51 as a rotating cylindrical body, to rotate the entirety of the helically shaped portion 51, the present invention is not limited thereto.

Aside from the above-described embodiment, the rotating driving force of the motor may be transmitted to, for example, a middle portion of the helically shaped portion 51 to rotate the entirety of the helically shaped portion 51.

Alternatively, the rotating driving force may be transmitted to a distal end portion of the helically shaped portion 51 to rotate the entirety of the helically shaped portion 51.

Meanwhile, although in the above-described embodiment, the helically shaped portion 51 is configured by the coil 91 and the thin resin coat 92, the present invention is not limited thereto. For example, the thin resin coat 92 may be omitted to provide a simple configuration only by the coil 91 loosely wound.

Furthermore, although in the above-described embodiment, the coil 91 has a generally circular wire sectional shape as shown in FIGS. 2, 5, 10 and so on, the present invention is not limited thereto. The wire sectional shape may be formed in an ellipse or rectangle, for example.

### (Examples)

By the way, in the above-described embodiment, the lead angle setting of the coil 91 of the helically shaped portion 51 defines the propulsive force generated when the helically shaped portion 51 rotates to advance and retreat the distal end portion of the endoscope insertion portion in the body cavity.

Here, note that the lead angle of the coil 91 refers to an angle defined by the thread of the coil 91 and a plane which passes through one point on the thread and is orthogonal to a longitudinally directed axis of the helically shaped portion 51.

In order to generate a propulsive force capable of advancing and retreating the distal end portion of endoscope insertion portion in the body cavity without any problem to perform a normal endoscopy, the lead angle of the coil 91 is preferably set to a value in, for example, a range from not less than 9 degrees to not more than 15 degrees as mentioned above.

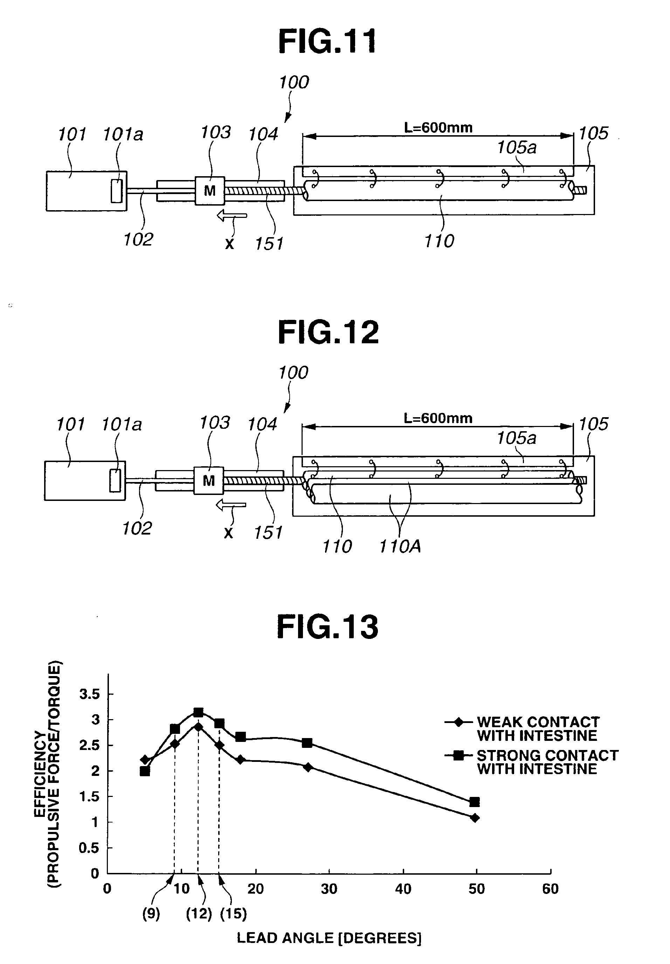

This can be verified by using an experimental device as shown in FIG. 11.

FIGS. 11 and 12 are each a general configuration view showing a schematic configuration of a measuring apparatus for measuring propulsive force of the helically shaped portion in an embodiment of the present invention. FIGS. 11 and 12 show cases where contact between the coil and the subject (intestine) is set to be weak and strong, respectively. Also, FIGS. 11 and 12 show the measuring apparatus as viewed from the upside thereof.

A measuring apparatus 100 is mainly configured by a device to be inspected that corresponds to the helically shaped portion and the driving motor for driving the helically shaped portion in the above-described embodiment; a push-pull gauge 101 including a sensor 101 a for measuring pushing and pulling forces; a coupling member 102 for coupling the push-pull gauge 101 and a part (a motor 103 to be described later) of the device to be inspected; a slide guide 104 for mounting thereon the part (the motor 103) of the device to be inspected and guiding the part's movement in a predetermined direction; and a table 105 including a fixing portion 105a for fixedly mounting thereon a part (a coil shaft 151) of the device to be inspected and a subject 110.

The above-described device to be inspected is a device corresponding to the helically shaped portion 51 and the motor in the motor box 16 in above-described embodiment, and is configured by the coil shaft 151 similarly configured to the above-described helically shaped portion 51 and the motor 103 coupled with a proximal end portion of the coil shaft 151 and for applying rotation force to the coil shaft 151.

Note that the coil shaft 151 of the device to be inspected is made of a cylindrically shaped aluminum pipe as a core material with an outer circumferential surface wound thereon with a silicone tube to form a helical shape.

When the device to be inspected is operated with the coil shaft 151 inserted into the subject 110, rotation force of the motor 103 is transmitted to the coil shaft 151, which is rotated about an axis thereof. As a result, propulsive force of the device to be inspected is obtained due to contact action between the helically shaped portion on the outer circumferential surface of the coil shaft 151 and the subject 110.

Note that in the measurement experiment performed using the experimental device, a pig intestine is used as the subject 110 into which the coil shaft 151 is to be inserted.

To measure the propulsive force of the coil shaft 151 (the helically shaped portion) of the device to be inspected using the experimental device configured as mentioned above, following steps are performed.

First, the subject 110 is linearly fixed on the table 105 using the fixing portion 105a of the table 105. Then, the coil shaft 151 is inserted into a tube cavity of the subject 110. Further, the motor 103 of the device to be inspected is mounted on the slide guide 104.

Note that range of contact between the helically shaped portion of the coil shaft 151 and the subject 110 when the coil shaft 151 is inserted into the subject 110 is set to be 600 mm, as shown in FIG. 11. At this time, the helically shaped portion formed on and along the outer circumferential surface of the shaft 151 is in contact with an internal wall surface of the subject 110. The insertion portion main body 10 requires the maximum propulsive force when passing a flexion as a boundary between the sigmoid colon insertion into which is difficult and the descending colon. Therefore, it is preferable that the helically shaped portion 51 occupies the entire region of contact with the intestine when the insertion portion main body 10 passes the flexion as a boundary between the sigmoid colon and the descending colon. Accordingly, assuming that length from the anus to the flexion as the boundary between the sigmoid colon and the descending colon is 600 mm, as generally said, the helically shaped portion 151 is required to have a length of at least 600 mm.

When power is supplied to the motor 103 in this state, the motor 103 starts driving and rotating in a predetermined direction, which also causes the coil shaft 151 to rotate in the same direction as that of a rotation shaft of the motor 103. At this time, because the coil shaft 151 is rotated with the helically shaped portion of the coil shaft 151 in contact with the internal wall surface of the subject 110, the coil shaft 151 generates propulsive force in, for example, a direction of an arrow X in FIG. 11. Therefore, the coil shaft 151 pushes the motor 103 in the same direction. The resultant pushing force is inputted to the sensor 101 a via the coupling member 102. In this manner, the propulsive force (pushing force) of the coil shaft 151 is measured by the push-pull gauge 101. At this time, motor torque value is also calculated based on a current value required to drive and rotate the motor 103.

Then, based on a measured propulsive force value and a calculated motor torque value, an efficiency value of the device to be inspected (helically shaped portion) is calculated. Here, the efficiency value signifies a ratio between the measure propulsive force value and the motor torque value (propulsive force/torque), that is, a propulsive force (g) per unit torque (g·cm).

It is understood that a larger efficiency value provides a more preferable condition, because it is preferred that the helically shaped portion to be applied to the endoscope insertion portion for endoscopy achieves reduction of motor torque while aiming for improving the propulsive force.

On the other hand, it is considered that different propulsive force and torque values may be obtained depending on state of contact between the helically shaped portion on the outer circumferential surface of the coil shaft 15 and the internal wall surface of the subject 110.

Accordingly, in the experiment performed using the experimental device, also measured is a propulsive force in a case where the state of contact between the coil shaft 151 and the subject 110 is set with a larger force as shown in FIG. 12 compared to the state shown in FIG. 11. FIG. 12 shows an exemplary setting configuration for this case. That is, on the subject 110 with the coil shaft 151 inserted therein in the state shown in FIG. 11, two subjects 110A are superposedly mounted. At this time, the coil shaft 151 is subject to a weight burden of the additional two pieces of subject 110A compared to the state shown in FIG. 11. Therefore, the coil shaft 151 at this time is under a larger burden of force compared to the state shown in FIG 11. Providing such a state patterns a state where the intestine is applied with an abdominal pressure or the like when, for example, an endoscope is inserted into a subject such as an intestine.

The coil shaft 151 applied for measuring the propulsive force of the coil shaft 151 using the above-described experimental device has a diameter of 8 mm, with the helically shaped portion having a wire diameter of 1 mm. The helically shaped portion of the coil shaft 151 is in single winding. Also, seven types of the coil shaft 151 are prepared respectively having lead angles set to 5, 9, 12, 15, 18, 27 and 50 degrees.

Results of thus performed experiments are shown in FIGS. 13 to 15. FIGS. 13 to 15 show, of the experimental results, changes in efficiency value, propulsive force value (g), and motor torque value (g·cm), respectively, when the lead angle is changed.

First, as shown in FIG. 13, efficiency values obtained by changing the lead angle tend to indicate high values when the lead angle is in the range from not less than 9 degrees to not more than 15 degrees. This tendency holds true irrespective of whether the contact between the coil shaft 151 and the intestine which is the subject 110 is strong or weak.

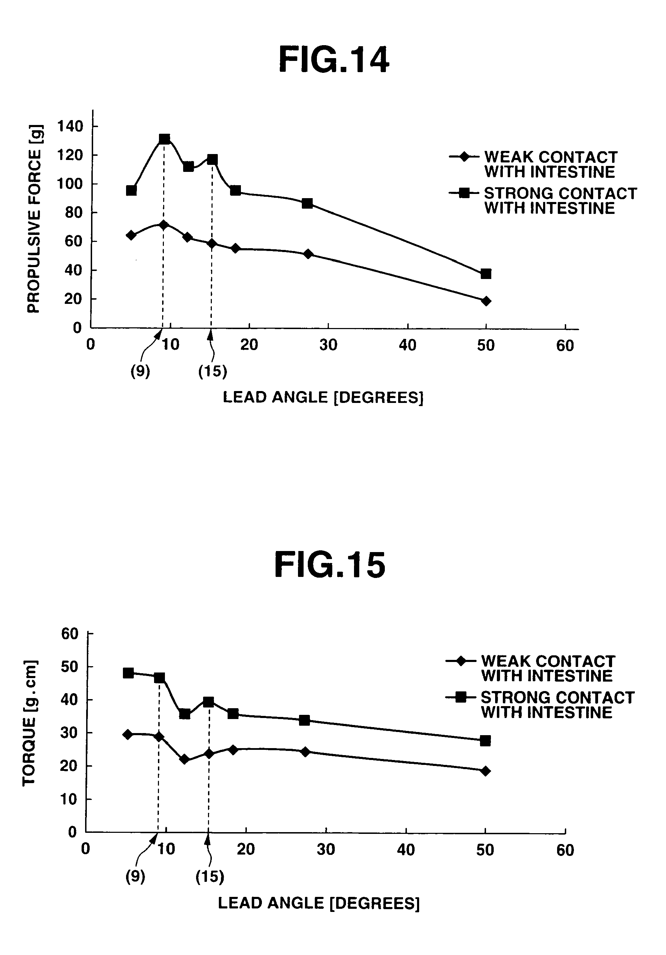

Also, as shown in FIG. 14, propulsive force values measured by changing the lead angle tend to indicate high values when the lead angle is in the range from not less than 9 degrees to not more than 15 degrees. The propulsive force value at this time indicates not smaller than about 100 grams as shown in the drawing. This propulsive force value is considered to be a sufficient for advancing and retreating the endoscope insertion portion in the body cavity in an endoscopy.

Although lower motor torque values are more preferable, in order to generate a larger propulsive force, motor torque value is made higher because a contact between the coil shaft 151 and the intestine is made stronger. Thus, as shown in FIG. 15, when the lead angle is in the range from not less than 9 degrees to not more than 15 degrees, the motor torque value is shown to be relatively high, with a peak value present at a point where the lead angle is 5 degrees, while motor torque values in the lead angle range from not less than 9 degrees to not more than 15 degrees are lower than the peak value.

From the above-described experimental results, it is deemed preferable to set the lead angle of the coil 91 of the helically shaped portion 51 in the above-described embodiment in an angle range from not less than 9 degrees to not more than 15 degrees.

Note that the present invention is not limited to the above-described embodiment, and various modifications and applications are of course possible without departing from the spirit of the invention.

It is apparent that, in the present invention, embodiments differing in a wide range may be configured based on the present invention without departing from the spirit and scope of the invention. The present invention shall not be limited by any specific embodiment thereof except by the appending claims.

## Claims

1. An endoscope comprising:
a flexible elongate endoscope insertion portion insertable into a subject's body; and
a flexible propulsive force generating portion rotatable on an outer circumferential side of the endoscope insertion portion and having a helically shaped portion on an outer circumferential surface of the flexible propulsive force generating portion,
wherein the helically shaped portion has a lead angle that is set to be in a range from not less than 9 degrees to not more than 15 degrees.

2. The endoscope according to claim 1, wherein the helically shaped portion has a length of not less than 600 mm.

3. An endoscope system comprising:
an endoscope including a flexible elongate endoscope insertion portion insertable into a subject's body; and a flexible propulsive force generating portion rotatable on an outer circumferential side of the endoscope insertion portion and having a helically shaped portion on an outer circumferential surface of the flexible propulsive force generating portion, the helically shaped portion having lead angle that is set to be in a range from not less than 9 degrees to not more than 15 degree; and
a rotation device for rotating the propulsive force generating portion about a longitudinal axis.
